**Europäisches Patentamt**

**European Patent Office**

**Office européen des brevets**

(11) Numéro de publication : **0 403 392 B1**

(12) # FASCICULE DE BREVET EUROPEEN

(45) Date de publication du fascicule du brevet :
**27.07.94 Bulletin 94/30**

(51) Int. Cl.$^5$ : **C13K 13/00, C12P 19/02**

(21) Numéro de dépôt : **90401661.5**

(22) Date de dépôt : **14.06.90**

(54) **Nouveau procédé de fabrication du xylose.**

(30) Priorité : **16.06.89 FR 8908046**

(43) Date de publication de la demande :
**19.12.90 Bulletin 90/51**

(45) Mention de la délivrance du brevet :
**27.07.94 Bulletin 94/30**

(84) Etats contractants désignés :
**AT BE CH DE DK ES GB IT LI NL SE**

(56) Documents cités :
**FR-A- 1 518 510**
**US-A- 4 394 178**
**JOURNAL OF CHROMATOGRAPHY, vol. 437,**
**1988, pages 387-397; J.O. BAKER et**
**al.:"Degradation of ketoses during aqueous**
**high-performance liquid chromatographyon**
**lead-form cation-exchange resins"**

(56) Documents cités :
**Journal of fermentation technology, vol. 64, n**
**4, 1985, pages 331-335 S. Ohmomo et al**
**"Biotransformation of D-xylulose to D-xylose**
**by immobilized enzyme prepared from strep-**
**tomyces flavorirensis"**

(73) Titulaire : **Roquette Frères**
**F-62136 Lestrem (FR)**

(72) Inventeur : **Leleu, Jean-Bernard**
**22 Place du 8 Mai**
**F-62136 Lestrem (FR)**
Inventeur : **Duflot, Pierrick**
**155 Rue des Mioches**
**F-62136 Lestrem (FR)**
Inventeur : **Caboche, Jean-Jacques**
**1327 Rue du Moulin Masclés**
**F-62400 Béthune (FR)**

(74) Mandataire : **Koch, Gustave et al**
**Cabinet PLASSERAUD**
**84, rue d'Amsterdam**
**F-75009 Paris (FR)**

**EP 0 403 392 B1**

## Description

L'invention a pour objet un procédé de fabrication du D-xylose.

Il est connu de préparer le D-xylose à partir de matières premières telles que le bois de bouleau, les rafles de maïs, les coques de graines ou d'amandes.

Par hydrolyse acide de ces matières premières, dans des conditions extrêmes de température et de pression, on décompose les xylans --polymères du D-xylose-- en xylose dont l'application essentielle est la fabrication --par hydrogénation-- du xylitol.

Ce procédé souffre toutefois de nombreux inconvénients dont les principaux sont:

- la faible teneur en xylans des matières premières, ce qui se traduit par de faibles rendements en xylose (de 8 à 15% en poids de la matière première mise en oeuvre) et par la génération d'une quantité considérable de sous-produits pour lesquels il est difficile, voire illusoire de trouver une revalorisation et dont la mise au rebut s'avère très polluante,
- la présence dans les hydrolysats de ces matières premières d'autres sucres que le xylose, à savoir ceux du groupe comprenant le glucose, le mannose, le galactose et l'arabinose dont les propriétés physiques sont proches de celles du D-xylose (les propriétés physiques des équivalents hydrogénés de ces sucres sont proches de celles du xylitol), ce qui rend très difficile la séparation du D-xylose (et, le cas échéant, du xylitol); or, la présence du galactitol, qui se trouve normalement dans les hydrolysats de bois hydrogénés et qui cristallise en même temps que le xylitol lorsqu'on concentre les sirops, est indésirable dès lors que le xylitol est destiné à l'alimentation car ledit galactitol provoque la cataracte.

Le brevet français N° 2.009.331, qui décrit un procédé de fabrication de D-arabitol par fermentation notamment de glucose, indique que le D-arabitol est une matière première importante pour préparer le D-xylose en passant par le D-xylulose; ce brevet reste toutefois muet sur les moyens susceptibles d'être mis en oeuvre pour transformer le D-xylulose en D-xylose, seul pentose (avec son isomère optique le L-xylose) fournissant 100% de xylitol par hydrogénation.

Il est vrai que l'isomérisation partielle par voie chimique du d-xylulose en d-xylose peut être envisagé mais elle présente l'inconvénient de faire intervenir des solvants dangereux.

L'isomérisation enzymatique semble donc préférable. D'ailleurs, HOCHSTER et WATSON (National Research Council n° 3105 Ottawa, Canada) ont réalisé à l'aide d'enzymes capables d'effectuer la conversion du D-xylose en D-xylulose et vice-versa, à titre expérimental, l'isomérisation du D-xylulose en D-xylose, mais ceci sans isoler le D-xylose à l'état de haute pureté et dans des conditions de température et surtout de concentration totalement incompatibles avec une mise en oeuvre à l'échelle industrielle.

De plus, la conversion effectuée à l'aide de ces enzymes, tout comme l'isomérisation chimique, n'est que partielle; ces isomérisations ne permettent d'obtenir le D-xylose qu'avec un rendement maximum de 75% et 25% du D-xylulose sont ainsi irrémédiablement perdus puisque non convertis en D-xylose.

Pour séparer le D-xylose du D-xylulose, il a été proposé (brevet français N° 2.117.558) de chromatographier un sirop contenant ces deux sucres sur une résine anionique chargée sous forme bisulfite. La séparation des sucres est bonne dans ces conditions; mais l'inconvénient de cette technique réside dans le fait qu'une partie du D-xylose se lie irréversiblement à la résine et qu'il est difficile de réaliser plus de cinq cycles de séparation consécutifs sans assister à une baisse notable des performances [S.P. Olivier et P.J. du Toit, Biotechnology and bioengineering, vol. XXVIII, pages 684-699 (1986)].

Il a également été proposé, dès 1972, par la demande de brevet japonais N° 47-13707, de préparer du xylitol sans passer par le xylose en hydrogénant directement le D-xylulose obtenu par double fermentation aérobie à partir du glucose. L'inconvénient de ce procédé réside dans le fait que ladite hydrogénation ne fournit que 50% de xylitol en même temps que 50% de D-arabitol, ce dernier étant, lorsqu'il est présent en de telles proportions, difficile à séparer du xylitol.

Par conséquent, il n'existe toujours pas de procédé permettant de produire à l'échelle industrielle du D-xylose ou du xylitol avec une pureté et un rendement suffisants à partir du xylulose, qui lui-même ne peut être obtenu qu'avec un rendement faible, voisin de 40%, à partir du D-glucose en passant par le D-arabitol.

L'invention a donc pour but, surtout, de fournir un procédé de préparation du D-xylose à partir du D-xylulose, lui-même obtenu à partir du D-glucose, propre à aboutir au D-xylose avec un rendement et une pureté suffisamment élevés pour que l'industriel puisse s'accomoder des nombreuses étapes conduisant depuis le D-glucose jusqu'au produit final recherché.

Et c'est à ce problème posé depuis près de vingt ans que la Société Demanderesse a eu le mérite d'apporter une solution en trouvant qu'il était possible d'isoler à l'état de haute pureté du D-xylose à partir d'un mélange de D-xylose et de D-xylulose en ayant recours à une chromatographie sur résines ou zéolithes cationiques, les résines cationiques étant préférées et constituées notamment par celles qui sont utilisées pour la séparation chromatographique du glucose et du fructose.

2

Il s'ensuit que le procédé de fabrication du D-xylose conforme à l'invention est donc caractérisé par le fait que:

- dans une première étape, un sirop de D-xylulose est soumis à une isomérisation enzymatique fournissant un mélange de D-xylose et de D-xylulose,
- dans une deuxième étape, le susdit mélange est soumis à un traitement chromatographique conduisant à au moins deux fractions dont l'une est enrichie fortement en D-xylose (fraction $X_1$) et dont l'autre est enrichie fortement en D-xylulose (fraction $X_2$),
- dans une troisième étape, la fraction $X_2$ est recyclée à l'étape d'isomérisation,

le D-xylose étant récupéré à partir de la fraction $X_1$, celle-ci pouvant également être soumise directement à une étape d'hydrogénation.

Le D-xylulose peut être obtenu de façon connue en elle-même et notamment par oxydation microbiologique du D-arabitol qui en constitue d'ailleurs le procédé d'obtention préféré, ledit D-arabitol étant obtenu par fermentation aérobie du D-glucose.

Selon un mode de réalisation avantageux, le procédé de fabrication du D-xylose conforme à l'invention est donc caractérisé par le fait que le D-xylulose de départ est préparé par une succession d'étapes comprenant:

- une fermentation aérobie d'un sirop de D-glucose à l'aide d'un microorganisme osmophile du genre Pichia, transformant le D-glucose en D-arabitol,
- une fermentation aérobie du sirop de D-arabitol à l'aide d'un microorganisme producteur d'alcool déshydrogénase, du genre Acetobacter, Gluconobacter ou Klebsielle, propre à transformer le D-arabitol en D-xylulose,
- une isomérisation du sirop de D-xylulose sous l'action de la glucose isomérase ou de la xylose isomérase, en un sirop riche en D-xylose.

La fermentation aérobie du glucose peut être replacée par une variante consistant à passer par l'oxydation du glucose en acide gluconique qui, sous sa forme de sel de calcium, peut être décarboxylé selon la méthode dite de RUFF pour donner le D-arabinose (cf. le brevet américain N° 3.755.294). Le D-arabinose est ensuite hydrogéné de manière connue en elle-même pour fournir le D-arabitol.

En dépit de sa complexité apparente, le procédé conforme à l'invention permet, grâce à la combinaison particulière de ses étapes constitutives, d'obtenir du D-xylose avec un rendement supérieur à 30% par rapport au D-glucose de départ qui est une matière première abondante et de faible prix.

Par rapport aux procédés de l'art antérieur,
- les quantités de matière première à mettre en oeuvre et
- la pollution ainsi que le volume de sous-produits générés par la fabrication du D-xylose et par la suite du xylitol

sont fortement diminués.

D'autres avantages résident dans le fait
- que les problèmes de logistique concernant la collecte de la matière première, le D-glucose, n'existent pas,
- que le traitement de cette matière première peut être réalisé dans des équipements classiques qui n'ont pas à résister à des températures et pressions extrêmement élevées ni à des milieux corrosifs,
- que le D-xylose est obtenu exempt de galactose, le xylitol obtenu par hydrogénation ne contenant donc pas de galactitol et pouvant, par conséquent, être utilisé dans l'alimentation.

L'invention sera encore mieux comprise à l'aide du complément de description qui suit, des exemples non limitatifs et du dessin ci-annexé, lesdits compléments de description, exemples et dessin étant relatifs à des modes de réalisation avantageux.

Dans le susdit dessin,
- la figure 1 montre schématiquement le déroulement du procédé conforme à l'invention,
- les figures 2 à 4 montrent schématiquement des parties d'une installation propres à la mise en oeuvre dudit procédé.

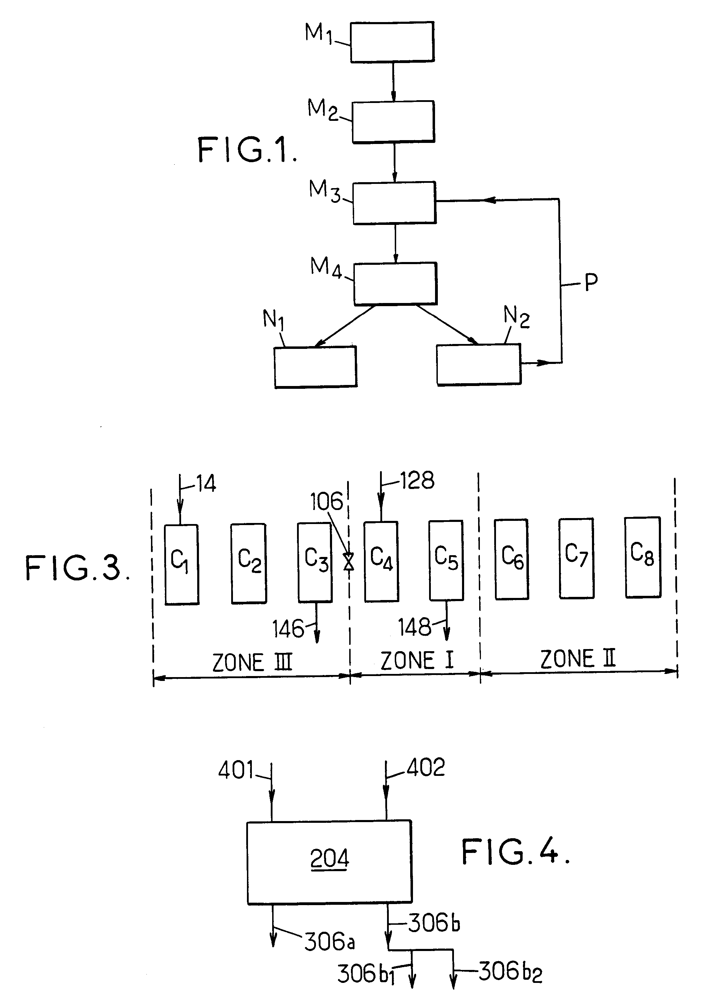

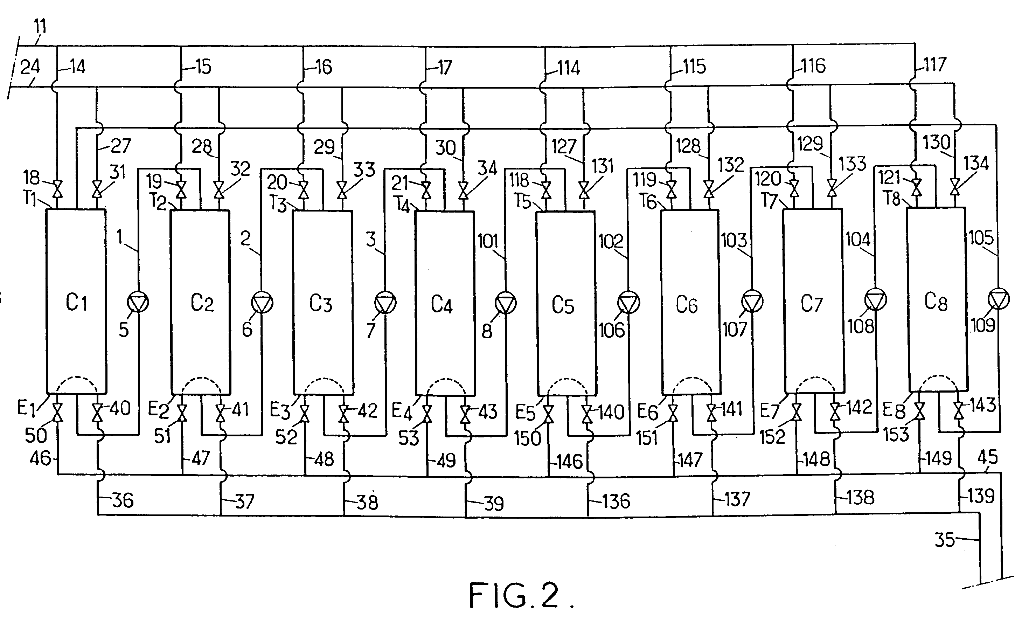

Sur la figure 1 du dessin ci-annexé, on a représenté schématiquement le déroulement du susdit procédé, à savoir:
- la transformation du D-glucose en D-arabitol en $M_1$,
- la transformation du D-arabitol en D-xylulose en $M_2$,
- l'isomérisation du D-xylulose en $M_3$,
- le traitement chromatographique du sirop isomérisé en $M_4$,
- la récupération d'un sirop riche en D-xylose en $N_1$ et celle d'un sirop riche en D-xylulose en $N_2$,
- le recyclage depuis $N_2$ vers $M_3$ par une canalisation P du sirop riche en D-xylulose.

Pour la fermentation du glucose, on peut avoir recours à un milieu de culture présentant la composition

3

suivante:

- dextrose       150 à 200 g/l
- azote organique (sous forme de corn-steep ou d'extrait de levure       2 à 4 g/l (N x 6,25)
- $KH_2PO_4$       1 à 3 g/l
- $MgSO_4$, $7H_2O$       1 à 2 g/l

et qui est introduit dans un fermenteur, stérilisé puis inoculé à l'aide de 10% environ d'une culture de 24 heures d'un microorganisme du genre Pichia, par exemple de la souche Pichia Ohmeri n° 20.209 conservée à l'A.T.C.C. (ou d'une souche de Pichia farinosa), cette culture ayant été réalisée sur un milieu constitué par exemple comme suit:

- glucose       50 g/l
- extrait de levure       10 g/l
- $KH_2PO_4$       3 g/l
- $MgSO_4$, $7H_2O$       1 g/l.

La fermentation est poursuivie à une température voisine de 30°C durant 80 à 100 heures sous une aération correspondant à 1 à 1,5 volume d'air/volume de culture/ minute, et à un pH compris entre 4 et 6, de préférence voisin de 4,5, avantageusement maintenu par de l'ammoniaque, ce grâce à quoi on obtient généralement une teneur en arabitol de 65 à 90 g/l, cet arabitol représentant de 70 à 85% des matières sucrées présentes dans le milieu de culture au terme de cette fermentation.

Le rendement en arabitol par rapport au glucose mis en oeuvre est d'environ 40 à 50%.

Le contenu entier du fermenteur (moût de fermentation riche en arabitol) est alors stérilisé de manière à détruire la levure; il est ensuite ensemencé pour l'étape de fermentation du D-arabitol par un inoculum (10% environ) d'une culture d'acetobacter suboxydans cultivé pendant 20 heures environ sur un milieu ayant la constitution suivante:

- arabitol       50 g/l
- sorbitol       2 g/l
- extrait de levure       2 g/l
- $KH_2PO_4$       0,2 g/l
- $MgSO_4$, $7H_2O$       0,2 g/l
- $CaCO_3$       5 g/l.

Il est avantageux de soumettre le moût de fermentation riche en arabitol à une purification par centrifugation ou filtration avant ensemencement par l'acetobacter.

La fermentation du D-arabitol est poursuivie sans ajouter d'autres substances nutritives à une température de 20 à 40°C, sous une aération correspondant à 1 à 1,5 volume d'air/volume de culture/minute, à un pH de 4,0 à 6,0 et durant un temps généralement compris entre 24 et 48 heures, terme au bout duquel on obtient un moût riche en D-xylulose, ce D-xylulose représentant de 70 à 85% des matières sucrées présentes au terme de cette deuxième fermentation.

Les impuretés sucrées présentes à ce niveau sont principalement constituées de D-arabitol ayant échappé à l'oxydation par l'acetobacter et de xylitol formé de façon parallèle à l'arabitol au cours de la première fermentation; on trouve également parmi ces impuretés des sucres comme le lyxose et quelques traces de glucose ou de saccharides divers qui étaient présents à l'état d'impuretés dans le dextrose ayant servi de matière première.

La composition glucidique du moût de fermentation obtenu à l'issue de l'étape de fermentation du D-arabitol est comme suit:

- xylulose       70 à 85%
- arabitol       5 à 15%
- xylitol       1 à 5%
- saccharides divers       5 à 10%.

Ce moût de fermentation peut être purifié de manière connue en elle-même (par filtration, décoloration au charbon actif et déminéralisation) puis concentré avant d'être soumis à l'étape d'isomérisation.

Il peut être obligatoire de recourir à la susdite purification si l'isomérisation est effectuée en continu à l'aide d'une enzyme immobilisée dans un réacteur à effet piston; cette purification est superflue dans le cas où l'on procède à une isomérisation en lot à enzyme perdue et de façon discontinue.

On peut utiliser pour l'étape d'isomérisation une xylose isomérase commerciale du type de celles mises en oeuvre pour la fabrication des sirops de maïs à haute teneur en fructose, à savoir par exemple:

- celle qui est connue sous la marque SPEZYME et qui est commercialisée par Suomen Sokeri,
- celle qui est connue sous la marque LYSASE GI 2000 et qui est fabriquée par la Société Demanderesse (brevet français N° 2.353.562).

De préférence, la quantité d'enzyme mise en oeuvre est telle que l'équilibre de la réaction soit atteint en 4 à 48 heures; la présence d'un agent protecteur de l'enzyme tel que le bisulfite de sodium et/ou un sel de magnésium est souhaitable.

L'isomérisation est conduite à une température de 40 à 80°C et à un pH compris entre 6,0 et 8,5.

D'une façon générale, les paramètres de l'étape d'isomérisation sont choisis de façon telle que celle-ci conduit à un sirop présentant une teneur en D-xylose supérieure à 53%.

A l'issue de l'étape d'isomérisation, la composition glucidique du sirop isomérisé obtenu est généralement la suivante:

- xylose        53 à 64%
- xylulose        17 à 22%
- arabitol        5 à 15%
- xylitol        1 à 5%
- saccharides divers        5 à 10%.

Il a été constaté avec surprise que la présence du xylitol formé de façon parallèle à l'arabitol lors de la fermentation du glucose et non transformé au moment de la fermentation du D-arabitol ne perturbe pas le fonctionnement de la xylose isomérase puisque les taux maximum de xylose sont identiques (voisins de 75%) aux taux obtenus antérieurement dans l'isomérisation du xylose pur.

Ce fait est d'autant plus inattendu qu'en 1988, IZUMORI et TUZAKI, "J. Ferment. Technol.", vol. 66, n°1, 33-36 (1988), ont écrit qu'il semble bien que le xylitol est un inhibiteur compétitif de la xylose isomérase; sa seule présence dans les sirops soumis à isomérisation aurait dû fortement entraver la réaction d'isomérisation, rendant par là-même impossible la réalisation du procédé selon l'invention.

Le sirop riche en xylose obtenu après isomérisation peut être purifié par déminéralisation et est ensuite soumis à l'étape de fractionnement chromatographique.

Cette étape de fractionnement chromatographique peut être effectuée de manière connue en elle-même, de façon discontinue ou continue (lit mobile simulé), sur des adsorbants du type résines cationiques fortement acides, de préférence chargées en ions alcalins ou alcalino-terreux, ou encore du type zéolithes cationiques chargées en ions $NH_4^+$, $Na^+$, $K^+$ et $Ca^{2+}$, $Ba^{2+}$ et autres.

Des exemples de tels procédés de séparation chromatographique sont donnés dans les brevets US 3.044.904, US 3.416.961, US 3.692.582, FR 2.391.754, FR 2.099.336, US 2.985.589, US 4.024.331, US 4.226.977, US 4.293.346, US 4.157.267, US 4.182.633, US 4.332.623, US 4.405.445, US 4.412.866 et US 4.422.881.

Suivant un mode de réalisation préféré, l'étape de séparation chromatographique est effectuée par mise en oeuvre du procédé et de l'appareillage décrits dans le brevet US 4.422.881 et son correspondant français N° 2.454.830 dont la Société Demanderesse est titulaire.

Quel que soit le procédé de séparation chromatographique retenu, on a recours, à titre d'adsorbant, à un matériau cationique, de préférence à une résine cationique forte, cette résine étant plus préférentiellement encore employée sous forme ionique calcium et ayant un taux de divinylbenzène d'environ 4 à 10%.

Le choix des paramètres de l'étape de chromatographie, dont notamment:

- le débit d'élution,
- le débit d'alimentation en sirop isomérisé,
- le débit d'extraction de la fraction enrichie en xylose,
- la composition des zones de désorption, d'adsorption et d'enrichissement,

se trouve expliqué et illustré dans l'exemple.

Ce choix est fait de telle sorte que la fraction $X_1$ présente une richesse en D-xylose, les pourcentages étant exprimés en poids sur matières sèches:

- de 60 à 95%,
- de préférence, de 75 à 90% et, plus préférentiellement encore, de 80 à 85%,

et une teneur en D-xylulose inférieure à 25% et, de préférence, inférieure à 15%.

Pour aboutir à ce résultat, on choisit lesdits paramètres comme suit lorsque l'étape de chromatographie est effectuée par mise en oeuvre du procédé et de l'appareillage décrits dans le brevet US 4..422.811 et que l'adsorbant utilisé est une résine cationique de faible granulométrie, réticulée à 6% de divinylbenzène et utilisée sous forme calcium:

- débit d'élution de 125 à 500 l/h/m³ d'adsorbant,
- débit d'alimentation en sirop isomérisé de 15 à 60 l/h/m³ d'adsorbant,
- débit d'extraction de la fraction enrichie en xylose de 30 à 120 l/h/m³ d'adsorbant.

L'étape de chromatographie conduit par ailleurs à l'obtention concomitante d'une fraction $X_2$ fortement enrichie en xylulose et à celle d'une fraction $X_3$ composée de produits très fortement adsorbés par le matériau cationique ou, au contraire, fortement exclus.

Parmi les produits fortement adsorbés par le matériau cationique, on trouve surtout le xylitol et l'arabitol et, parmi les produits fortement exclus, les divers saccharides.

La fraction $X_2$ fortement enrichie en D-xylulose a de préférence la composition suivante, les pourcentages étant exprimés en poids sur matières sèches:

- de 50 à 80% de xylulose,
- de 20 à 50% de xylose,
- de 0 à 5% d'arabitol et de xylitol.

Cette fraction $X_2$ fortement enrichie en D-xylulose est recyclée, conformément à l'invention, à l'étape d'isomérisation enzymatique.

C'est grâce à la mise en oeuvre conforme à l'invention des étapes de séparation chromatographique et de recyclage qu'il est devenu possible de transformer avec un rendement extrêmement élevé le D-xylulose en D-xylose tout en obtenant ce D-xylose à l'état de haute pureté, ce qui rend économiquement intéressante la fabrication du D-xylose à partir de D-glucose par l'intermédiaire du D-xylulose.

La fraction $X_3$ rassemblant les produits très fortement adsorbés par la résine ou, au contraire, très fortement exclus, est éliminée du système.

A partir de la fraction $X_1$ très riche en D-xylose, on récupère le D-xylose. Il est également possible de soumettre la fraction $X_1$ directement à une hydrogénation notamment catalytique.

Pour récupérer le D-xylose de la fraction $X_1$, il est possible:

- soit de concentrer le sirop pour séparer par cristallisation le D-xylose chimiquement pur, les eaux-mères épuisées pouvant alors avantageusement être recyclées vers l'étape chromatographique,
- soit de déshydrater dans sa totalité ledit sirop pour fournir du D-xylose de qualité technique.

Lorsqu'il est décidé d'hydrogéner directement la fraction $X_1$, on a recours aux conditions connues de l'art antérieur, notamment à des catalyseurs au ruthénium ou au nickel de Raney; cette hydrogénation directe est mise en oeuvre de préférence lorsque l'on désire fabriquer du xylitol; l'hydrogénation peut être effectuée avec un catalyseur au nickel de Raney, sous une pression d'hydrogène comprise entre 20 et 80 kg/cm² et à une température d'environ 80 à 130°C.

Le sirop de xylitol obtenu présente la composition suivante:

xylitol          87 à 97%
arabitol        3 à 13%.

Sa très grande richesse en xylitol permet de séparer le xylitol par cristallisation directement à partir de sa solution aqueuse avec un très fort rendement et à un très grand état de pureté et il est possible d'épuiser pratiquement les eaux-mères de la cristallisation en effectuant plusieurs jets consécutifs, comme il est indiqué par exemple dans le brevet français N° 2.202.069.

## EXEMPLE

### Etape de fermentation aérobie du D-glucose

Dans un fermenteur d'une capacité totale de 10 m³, on a introduit:

1200 kg de dextrose cristallisé monohydrate,
16 kg d'extrait de levure,
8 kg de $KH_2PO_4$,
8 kg de $MgSO_4$, $7H_2O$.

Après stérilisation du milieu de culture et refroidissement à 30°C, on a inoculé ce fermenteur à l'aide de 800 litres d'une préculture de Pichia Ohmeri ATCC 20.209 telle que décrite dans le brevet français N° 2.009.331, préculture âgée de 24 heures.

L'aération a été poursuivie durant toute la durée de la transformation du glucose en arabitol soit durant 90 heures avec un débit de 130 Nm³/heure et le pH a été contrôlé par addition d'ammoniaque à une valeur de 4,5.

### Etape de fermentation aérobie du D-arabitol

Cette première étape étant achevée, le contenu du fermenteur a été stérilisé; ensuite, sans addition d'autres ingrédients nutritifs et après refroidissement à une température de 30°C, il a été à nouveau ensemencé, mais cette fois à l'aide de 800 litres d'une préculture âgée de 24 heures d'acetobacter suboxydans cultivée sur un milieu de la composition suivante:

- arabitol          50 g/l
- sorbitol          2 g/l
- extrait de levure          2 g/l

- KH$_2$PO$_4$        0,2 g/l
- MgSO$_4$, 7 H$_2$O        0,2 g/l.

Au terme de la fermentation, le moût de culture a été filtré, décoloré sur charbon actif et déminéralisé sur résines échangeuses d'ions.

La composition glucidique de ce sirop purifié s'est révélée être la suivante:
- xylulose        80,7%
- arabitol        6,5%
- xylitol        3,8%
- saccharides divers        9 %.

Ce sirop a été obtenu avec un rendement de 48% par rapport à la matière sèche de glucose mis en oeuvre, ce qui correspond à un rendement de 40% de xylulose pur par rapport au glucose mis en oeuvre.

Etape d'isomérisation

Le sirop purifié obtenu après l'étape de fermentation aérobie du D-arabitol a été concentré à 45% de matières sèches, puis il a été introduit dans une cuve thermostatée à 55°C en présence de glucose isomérase de la marque SPEZYME commercialisée par Suomen Sokeri. La dose d'enzyme employée a été de 2 kg pour les 2 m$^3$ de sirop présents dans la cuve. Le pH de ce sirop a été réglé à 7,0 et la réaction d'isomérisation s'est déroulée durant 24 heures en présence également de 0,7 ml de NaHSO$_3$ en solution à 30% et de 1 g/l de MgSO$_4$, 7H$_2$O.

Au terme de cette étape, le sirop obtenu avait la composition suivante:
- xylose        60 %
- xylulose        20 %
- arabitol        6,5%
- xylitol        3,8%
- saccharides divers        9,7%.

La proportion de xylulose par rapport au xylose dans ce sirop est donc de 25%, ce qui est la valeur normale d'équilibre de l'enzyme lorsque l'on effectue l'isomérisation en partant de xylose cristallisé pur. On constate donc, comme il a déjà été dit, que le xylitol ne s'est pas comporté comme un inhibiteur compétitif de l'enzyme d'isomérisation.

Etape de fractionnement chromatographique

On procède au fractionnement du sirop isomérisé riche en xylose dans l'installation de séparation chromatographique continue dont les détails de la construction et du fonctionnement sont ceux décrits dans le brevet US 4.422.881 et dans le brevet correspondant français N° 2.454.830, ces détails n'étant repris ici que dans la mesure où la compréhension du texte l'exige.

Cette installation comprend, comme montré à la figure 2 du brevet américain (reprise ici en tant que figure 2, pour l'explicitation détaillée de laquelle on se reportera au brevet américain), huit colonnes ou étages C$_1$ à C$_8$ de 200 litres chacune, garnies d'adsorbant du type résines cationiques fortes souf forme calcium et de fine granulométrie (0,2 à 0,4 millimètre) du type C204-2078 de Duolite.

De par le calage des électrovannes, on établit dans cette installation une zone I de désorption de deux étages, une zone II d'adsorption de 3 étages et une zone III d'enrichissement et de séparation du xylose faiblement adsorbé et des xylitol et arabitol fortement adsorbés de 3 étages, comme montré figure 3 qui est une représentation schématique de l'installation selon la figure 2 et sur laquelle on n'a fait figurer que:
- les colonnes C$_1$ à C$_8$,
- le dispositif de fermeture, en l'occurrence l'électrovanne 106,
- les canalisations d'alimentation en sirop isomérisé riche en xylose à fractionner et en eau, montrées respectivement en 14 et 128, et
- la canalisation 148 d'extraction de sirop enrichi en xylulose (fraction X$_2$), d'une part, et la canalisation 146 d'extractions successivement de xylitol-arabitol (fraction X$_3$), de saccharides divers (fraction X$_3$) et de xylose (fraction X$_1$), d'autre part.

Le dispositif de fermeture 106 (notamment une électrovanne) maintient dans la configuration adoptée, une étanchéité totale entre, d'une part, la zone III, qui est une zone d'enrichissement à l'extrémité de laquelle sont donc récupérés successivement le reliquat de xylitol-arabitol fortement adsorbé, les saccharides divers, puis la fraction enrichie en xylose et, d'autre part, la zone I de désorption du xylulose, zone en tête de laquelle est introduite l'eau de désorption.

Ce dispositif de fermeture assure le sens du passage de la phase liquide sur l'adsorbant sélectif.

Une minuterie ajustée à 26'30" assure pour les débits indiqués ci-après une alimentation en eau au premier étage ou première colonne de la zone I de désorption suffisante pour effectuer la désorption de la totalité du xylulose, et une alimentation en un volume de sirop isomérisé riche en xylose compatible avec le volume d'adsorbant et sa capacité d'adsorption, de façon à obtenir un taux d'extraction de xylose au moins égal à 60% du xylose présent dans le sirop isomérisé et cela à une richesse au moins égale à 60% en xylose.

Les susdits taux d'extraction et puretés sont maintenus constants en ajustant le débit de la pompe d'extraction non montrée du xylulose adsorbé. La sortie des fractions "arabitol-xylitol-saccharides divers" (fraction $X_3$) puis "xylose enrichi" (fraction $X_1$) s'effectue à pression atmosphérique et son débit constant résulte de la différence entre les débits d'alimentation et le débit d'extraction.

Le sirop isomérisé riche en xylose qui est introduit dans l'installation en tête de la zone d'enrichissement et de séparation III, présente, comme indiqué plus haut, une teneur en matières sèches de 50%. La température à l'intérieur des colonnes de séparation est maintenue à environ 70°C.

La figure 4 montre schématiquement en 204 l'installation des figures 2 et 3, les mêmes références désignant les mêmes éléments pour les parties communes que dans la figure 1. L'installation de chromatographie 204 comporte une canalisation 306b par laquelle sont éliminés les excédents d'eau contenant une fraction importante d'arabitol-xylitol et la fraction saccharides divers (fraction $X_3$). Ces extraits sont à faible teneur en matières sèches et sortent par la canalisation 306b$_1$.

L'alimentation en eau s'effectue par une canalisation 401.

Les flèches portées sur les canalisations indiquent le sens de la circulation.

L'ensemble de chromatographie 204 fonctionne comme suit:

- le sirop isomérisé riche en xylose devant être soumis au fractionnement chromatographique est acheminé par la canalisation 401 à un débit de 52 litres/heure et présente une teneur en matières sèches de 50%,
- le xylose enrichi (fraction $X_1$) est récupéré par la canalisation 306b$_2$ avec un débit de 88,5 litres/heure, sa teneur moyenne en matières sèches étant de 23,3%,
- la quantité totale de liquides extraits par ailleurs de l'installation l'est avec un débit total de 344,5 litres/heure, se composant:
  - d'une part,
    * d'une fraction d'excédent d'eau, extraite par la canalisation 306b$_1$, contenant à faible concentration et haute pureté, du xylitol-arabitol puis, à faible concentration et à richesse élevée, les saccharides divers (fraction $X_3$), l'ensemble représentant un équivalent de 265,5 litres/heure, la teneur en matières sèches étant de 2,5%; ces fractions correspondent aux 20 premières minutes du cycle,
    * d'une fraction de xylose fortement enrichie (fraction $X_1$), d'un équivalent de 88,5 litres/heure acheminée par une canalisation 306b$_2$ vers une installation de purification non montrée, la teneur en matières sèches de cette fraction étant de 23,3%; cette fraction correspond à la dernière partie du cycle soit 6'30",
  - et, d'autre part, d'une fraction fortement enrichie en xylulose et très appauvrie en xylose (fraction $X_2$), extraite à un débit de 79 litres/heure par la canalisation 306a (fig. 4) correspondant à la canalisation 148 de la fig. 3.

Les tableaux 1 et 2 ci-dessous résument les conditions caractérisant le fonctionnement du dispositif de fractionnement chromatographique.

## TABLEAU 1

| Entrées chromatographiques | Sirop riche en xylose | Eau | Total |
|---|---|---|---|
| Débit | 52 1/h | 381 1/h | 433 1/h |
| Densité | 1,25 | | |
| Matières sèches | 50 % | | |
| Débit massique | 32 kg/h | | |
| Richesse en xylose | 60 % | | |
| Débit massique en xylose | 19,2 kg/h | | |
| Débit massique en xylulose | 6,4 kg/h | | |

Les effluents extraits de l'installation sont identifiés dans le tableau 2.

## TABLEAU 2

| Sorties chromatographiques | Fraction enrichie en xylose ($X_1$) | Fraction enrichie en xylulose ($X_2$) | Arabitol+xylitol + saccharides divers ($X_3$) | Total |
|---|---|---|---|---|
| Débit | 88,5 1/h | 79 1/h | 265,5 1/h | 433 1/h |
| Densité | 1,08 | 1,02 | 1,01 | |
| Matières sèches | 23,3% | 5,9% | 2,5% | |
| Débit massique | 20,7 kg/h | 4,7 kg/h | 6,6 kg/h | 32 kg/h |
| Richesse en xylose | 84% | 32% | 5% | |
| Débit massique en xylose | 17,4 kg/h | 1,5 kg/h | 0,3 kg/h | 19,2 kg/h |
| Teneur en xylulose | 15% | 68% | 0% | |
| Débit massique en xylulose | 3,2 kg/h | 3,2 kg/h | 0 | |

Ce résultat correspond à un taux d'extraction massique de xylose de 20,7/32 = 65% de sirop enrichi à 84% de xylose, ce qui représente un taux d'extraction du xylose de 17,4/19,2 = 90,6%, et à un taux d'extraction massique du xylulose égal à 4,7/32 = 15% de sirop enrichi à 68% de xylulose, ce qui représente un taux d'extraction du xylulose de 3,2/6,4 = 50%.

Il est à noter qu'à ce niveau, on aurait pu augmenter par exemple fortement ce taux d'extraction du xylulose en augmentant par exemple le débit d'extraction de la fraction ($X_2$), ce qui se serait traduit par une diminution du débit de sortie des fractions ($X_1$) et ($X_3$). Corrélativement, on aurait obtenu une fraction ($X_1$) encore plus riche en xylose mais avec un rendement d'extraction du xylose un peu plus faible. La fraction ($X_2$) aurait par contre été un peu moins riche en xylulose.

L'analyse de la fraction sirop de xylose enrichi ($X_1$) donne les résultats suivants:
- xylitol-arabitol          traces
- xylulose          15%
- xylose          84%

- saccharides divers          1%.

L'analyse de la fraction sirop de xylulose enrichi ($X_2$) donne les résultats suivants:
- xylitol-arabitol          traces
- xylulose          68%
- xylose          32%.

La fraction riche en xylose peut être soumise à cristallisation de manière connue en soi après l'avoir purifiée et concentrée. Le xylose cristallisé ainsi obtenu peut être hydrogéné de façon à former du xylitol.

Dans ce cas, après épuisement des eaux-mères en xylose, celles-ci sont avantageusement recyclées vers l'étape de fractionnement chromatographique de façon à en extraire pratiquement tout le xylose. C'est ainsi que cette fraction a été concentrée sous vide à une matière sèche de 75% en présence de cristaux de D-xylose puisqu'elle a été refroidie à une température de 20°C sous agitation et en 24 heures. La masse cristallisée a été essorée et lavée et on a obtenu le xylose avec un rendement de 50% en un seul jet. La pureté de ce xylose était de 98%. Les eaux-mères avaient une richesse en xylose d'environ 68% et peuvent donc être avantageusement recyclées vers l'étape de chromatographie de façon à en extraire pratiquement tout le xylose.

La fraction riche en xylose peut être également hydrogénée directement pour fournir un sirop riche en xylitol.

C'est ainsi que cette fraction a été hydrogénée à l'aide d'un catalyseur au nickel de Raney sous une pression d'hydrogène de 45 bars et à une température de 120°C et a fourni un sirop d'une richesse de 91% de xylitol.

La fraction $X_2$ issue de l'étape de chromatographie a été soumise par recyclage une nouvelle fois à l'étape d'isomérisation enzymatique et ce dans les mêmes conditions que celles déjà décrites ci-dessus à propos de l'isomérisation du moût de fermentation riche en xylulose.

On a obtenu alors un sirop isomérisé dont la composition était la suivante:
- xylitol-arabitol          traces
- xylulose          26%
- xylose          74%.

Ce sirop a été remélangé avec le sirop riche en xylose et a été soumis à nouveau au fractionnement chromatographique.

Il résulte des valeurs numériques indiquées dans cet exemple que, grâce au procédé conforme à l'invention, on obtient, à partir de 131,5 kg de matières sèches glucidiques issues de l'étape de fermentation du D-arabitol, une quantité de 100 kg de matières sèches de sirop de xylose à 84% de richesse, ce qui, en tenant compte d'un rendement de transformation du glucose en xylulose de 48%, se traduit par une mise en oeuvre de glucose de 274 kg.

On obtient donc avec le procédé de l'invention le D-xylose à l'état de sirop très riche avec un rendement de 36% par rapport au glucose mis en oeuvre.

## Revendications

1.  Procédé de fabrication du D-xylose caractérisé par le fait que:
    - dans une première étape, un sirop de D-xylulose est soumis à une isomérisation enzymatique fournissant un mélange de D-xylose et de D-xylulose,
    - dans une deuxième étape, le susdit mélange est soumis à un traitement chromatographique conduisant à au moins deux fractions dont l'une est enrichie fortement en D-xylose (fraction $X_1$) et dont l'autre est enrichie fortement en D-xylulose (fraction $X_2$),
    - dans une troisième étape, la fraction $X_2$ est recyclée à l'étape d'isomérisation,
    le D-xylose étant récupéré à partir de la fraction $X_1$, celle-ci pouvant également être soumise directement à une étape d'hydrogénation.

2.  Procédé selon la revendication 1, caractérisé par le fait que le D-xylulose de départ est préparé par une succession d'étapes comprenant:
    - une fermentation aérobie d'un sirop de D-glucose à l'aide d'un microorganisme osmophile du genre Pichia, transformant le D-glucose en D-arabitol,
    - une fermentation aérobie du sirop de D-arabitol à l'aide d'un microorganisme producteur d'alcool déshydrogénase, du genre Acetobacter, Gluconobacter ou Klebsielle, propre à transformer le D-arabitol en D-xylulose,
    - une isomérisation du sirop de D-xylulose sous l'action de la glucose isomérase ou de la xylose isomérase, en un sirop riche en D-xylose.

10

3. Procédé selon l'une des revendications 1 et 2, caractérisé par le fait que l'enzyme utilisée est une glucose-isomérase.

4. Procédé selon l'une des revendications 1 à 3, caractérisé par le fait que l'étape de fractionnement chromatographique est effectuée en ayant recours à des résines ou zéolithes cationiques.

5. Procédé selon la revendication 4, caractérisé par le fait que l'étape de fractionnement chromatographique est effectuée de façon discontinue ou continue (lit mobile simulé), sur des adsorbants du type résines cationiques fortement acides, de préférence chargées en ions alcalins ou alcalino-terreux, ou encore du type zéolithe cationique chargé en ions $NH_4^+$, $Na^+$, $K^+$ et $Ca^{2+}$, $Ba^{2+}$.

6. Procédé selon l'une des revendications 1 à 5, caractérisé par le fait que les paramètres de l'étape d'isomérisation enzymatique sont choisis de façon telle que celle-ci conduit à un sirop d'une teneur en D-xylose supérieure à 53%.

7. Procédé selon l'une des revendications 1 à 6, caractérisé par le fait que les paramètres de l'étape de fractionnement chromatographique sont choisis de telle sorte que l'on obtienne une fraction riche en xylose présentant une teneur en ce produit, les pourcentages étant exprimés en poids sur matières sèches:
   - de 60 à 95%,
   - de préférence, de 75 à 90% et, plus préférentiellement encore, de 80 à 85%,
   et une teneur en D-xylulose inférieure à 25% et, de préférence, inférieure à 15%.

8. Procédé selon l'une des revendications 1 à 7, caractérisé par le fait que les eaux-mères de cristallisation de xylose sont recyclées à l'étape de fractionnement chromatographique.

## Patentansprüche

1. Verfahren zur Herstellung von D-Xylose, dadurch gekennzeichnet, daß
   - in einer ersten Stufe ein Sirup von D-Xylulose einer enzymatischen Isomerisierung unterzogen wird, die eine Mischung von D-Xylose und D-Xylulose liefert,
   - in einer zweiten Stufe die obengenannte Mischung einer chromatographischen Behandlung unterzogen wird, die zu mindestens zwei Fraktionen führt, von denen eine stark mit D-Xylose (Fraktion $X_1$) und die andere stark mit D-Xylulose (Fraktion $X_2$) angereichert ist,
   - in einer dritten Stufe die Fraktion $X_2$ wieder der Stufe der Isomerisierung zugeführt wird,
   wobei die D-Xylose ausgehend von der Fraktion $X_1$ gewonnen wird, die auch direkt einer Hydrierungs-Stufe unterzogen werden kann.

2. Verfahren nach Anspruch 1, dadurch gekennzeichnet, daß die D-Xylulose als Ausgangsstoff durch eine Aufeinanderfolge von Stufen hergestellt wird, die umfassen:
   - eine aerobe Fermentation eines Sirups von D-Glucose mit Hilfe eines osmophilen Mikroorganismus der Gattung Pichia, der D-Glucose in D-Arabitol umwandelt,
   - eine aerobe Fermentation des Sirups von D-Arabitol mit Hilfe eines Alkohol-Dehydrogenase produzierenden Mikroorganismus der Gattung Acetobacter, Gluconobacter oder Klebsielle, der geeignet ist, D-Arabitol in D-Xylulose umzuwandeln,
   - eine Isomerisierung des Sirups von D-Xylulose unter der Einwirkung von Glucose-Isomerase oder von Xylose-Isomerase zu einem mit D-Xylose angereicherten Sirup.

3. Verfahren nach einem der Ansprüche 1 und 2, dadurch gekennzeichnet, daß das verwendete Enzym eine Glucose-Isomerase ist.

4. Verfahren nach einem der Ansprüche 1 bis 3, dadurch gekennzeichnet, daß die Stufe der chromatographischen Fraktionierung durchgeführt wird, indem man auf Harze oder kationische Zeolithe zurückgreift.

5. Verfahren nach Anspruch 4, dadurch gekennzeichnet, daß die Stufe der chromatographischen Fraktionierung in kontinuierlicher (simuliertes Bewegt-Bett) oder diskontinuierlicher Art und Weise auf Adsorbentien vom Typ stark saurer kationischer Harze, vorzugsweise beladen mit Alkali- oder Erdalkali-Ionen, oder auch vom Typ kationischer Zeolithe, beladen mit Ionen $NH_4^+$, $Na^+$, $K^+$ und $Ca^{2+}$, $Ba^{2+}$, durchgeführt wird.

6. Verfahren nach einem der Ansprüche 1 bis 5, dadurch gekennzeichnet, daß die Parameter der Stufe der enzymatischen Isomerisierung in der Weise gewählt werden, daß sie zu einem Sirup mit einem Gehalt an D-Xylose von höher als 53 % führt.

7. Verfahren nach einem der Ansprüche 1 bis 6, dadurch gekennzeichnet, daß die Parameter der Stufe der chromatographischen Fraktionierung in der Weise gewählt werden, daß man eine an Xylose angereicherte Fraktion erhält, die einen Gehalt an diesem Produkt, ausgedrückt in Gewichtsprozent Trockensubstanz von
   - 60 bis 95 %
   - vorzugsweise von 75 bis 90 %, und besonders bevorzugt von 80 bis 85 %,
   und einen Gehalt an D-Xylulose von unter 25 %, vorzugsweise unter 15 %, aufweist.

8. Verfahren nach einem der Ansprüche 1 bis 7, dadurch gekennzeichnet, daß die Mutterlaugen der Kristallisation von Xylose wieder der Stufe der chromatographischen Fraktionierung zugeführt werden.


## Claims

1. Process for the manufacture of D-xylose characterized by the fact that:
   - in a first step, a syrup of D-xylulose is subjected to enzymatic isomerization providing a mixture of D-xylose and D-xylulose,
   - in a second step, the abovesaid mixture is subjected to chromatographic treatment leading to at least two fractions of which one is highly enriched in D-xylose (fraction $X_1$) and of which the other is highly enriched in D-xylulose (fraction $X_2$),
   - in a third step, the fraction $X_2$ is recycled to the isomerization step,
   the D-xylose being recovered from the fraction $X_1$, the latter could also be subjected directly to a hydrogenation step.

2. Process according to claim 1, characterized by the fact that the starting D-xylulose is prepared by a succession of steps comprising:
   - an aerobic fermentation of a syrup of D-glucose by means of an osmophilic microorganism of the Pichia genus, converting the D-glucose into D-arabitol,
   - an aerobic fermentation of the D-arabitol syrup by means of a microorganism producing dehydrogenase alcohol, of Acetobacter, Gluconobacter or Klebsiella genus, suitable for converting the D-arabitol into D-xylulose,
   - an isomerization of the D-xylulose syrup under the action of glucose isomerase or of xylose isomerase into a syrup rich in D-xylose.

3. Process according to one of claims 1 and 2, characterized by the fact that the enzyme used is a glucose-isomerase.

4. Process according to one of claims 1 to 3, characterized by the fact that the chromatographic fractionation step is carried out by resorting to cationic resins or zeolites.

5. Process according to claim 4, characterized by the fact that the chromatographic fractionation step is carried out discontinuously or continuously (simulated moving bed), on adsorbents of the highly acid cationic resin type, preferably charged with alcaline or alcaline-earth ions, or again of the cationic zeolite type charged with $NH_4^+$, $Na^+$, $K^+$ and $Ca^{2+}$, $Ba^{2+}$ ions.

6. Process according to one of claims 1 to 5, characterized by the fact that the parameters of the enzymatic isomerization step are selected so that the latter results in a syrup with a content of D-xylose higher than 53%.

7. Process according to one of claims 1 to 6, characterized by the fact that the parameters of the chromatographic fractionation step are selected so that there is obtained a fraction rich in xylose having a content of this product, the percentages being expressed by weight on dry matter:
   - from 60 to 95%,
   - preferably, from 75 to 90% and, more preferably still, from 80 to 85%,
   and a content of D-xylulose less than 25% and, preferably, less than 15%.

8. Process according to one of claims 1 to 7, characterized by the fact that the mother-liquors from crystallization of xylose are recycled to the chromatographic fractionation step.

FIG.1.

$M_1$

$M_2$

$M_3$

$M_4$

$N_1$

$N_2$

P

FIG.3.

14

106

128

$C_1$ $C_2$ $C_3$ $C_4$ $C_5$ $C_6$ $C_7$ $C_8$

146

148

ZONE III

ZONE I

ZONE II

FIG.4.

401

402

204

306a

306b

306b$_1$

306b$_2$

14

FIG.2.